# EUROPEAN PATENT APPLICATION

(11) **EP 3 101 619 A1**
(43) Date of publication of application: **07.12.2016**
(21) Application number: 15810188.1
(22) Date of filing: 19.06.2015
(51) Int. Cl.: G06Q 50/24

(54) **DRUG PRESCRIPTION ASSISTANCE METHOD, COMPUTER PROGRAM FOR DRUG PRESCRIPTION ASSISTANCE, AND DRUG PRESCRIPTION ASSISTANCE DEVICE**

(30) Priority: 20.06.2014 JP 2014127317
(71) Applicant: Panasonic Healthcare Holdings Co., Ltd., Tokyo 105-8433 (JP)
(72) Inventor: MINEMURA Atsushi, Toon-shi Ehime 791-0395 (JP); OZAKI Nobuhiko, Toon-shi Ehime 791-0395 (JP); SUZUKI Tetsu, Toon-shi Ehime 791-0395 (JP)
(74) Representative: Grünecker Patent- und Rechtsanwälte PartG mbB
(86) International application number: PCT/JP2015/067804
(87) International publication number: WO 2015/194673

(57) **Abstract**

A drug prescription assistance method of a server (2) for controlling the allowability of accessing a dosage history via a communication network (N), wherein the server (2) executes the steps of: receiving user data and prescribed drug data from a user terminal (1); setting, when the user data and prescribed drug data are received, an accessibility identifier indicating the allowability of accessing, from a pharmacy terminal (4), the dosage history of a user corresponding to the user data out of a database in which an individual and the dosage history of the individual are correlated, to an identifier meaning that the access is allowed; receiving a processing-finished signal from the pharmacy terminal (4); and changing, when the processing-finished signal is received, the accessibility identifier to an identifier meaning that the access is not allowed.

## Description

### TECHNICAL FIELD

The present invention relates to a medicine prescription support method for controlling propriety of access to a medicine taking history through a communication network, a medicine prescription supporting computer program for causing a computer to execute steps of the medicine prescription support method for controlling propriety of access to a medicine taking history through a communication network so as to control the access to the medicine taking history, and a medicine prescription support apparatus for controlling propriety of access to a medicine taking history through a communication network.

### BACKGROUND ART

A patient who has received a prescription from a medical institution such as a hospital can submit the prescription to a pharmacy selected freely by the patient, and purchase medicines. When the patient purchases the medicines at the pharmacy, a pharmacist checks the prescription and dispenses the medicines. On that occasion, the pharmacist should take intake of other medicines or the like into consideration. It is desired that the pharmacist refers to a past medicine taking history of the patient. In recent years, there has been known a technique in which a database of medicine taking histories of patients are stored in a server so that a medicine taking history of each patient can be confirmed by access from a terminal placed in each pharmacy to the database of the medicine taking histories stored in the server through a communication network. Patient specification information such as a name of a patient is input from a terminal placed in a pharmacy for the sake of reference to a medicine taking history of the patient. The medicine taking history of the patient is then displayed on the terminal placed in the pharmacy (for example, see Patent Document 1).

### PRIOR ART DOCUMENT

### PATENT DOCUMENT

Patent Document 1: JP-A-2005-135207

### SUMMARY OF THE INVENTION

### PROBLEMS TO BE SOLVED BY THE INVENTION

Patients can select pharmacies freely and use various pharmacies. It is desired that a pharmacist receiving a prescription dispenses medicines after checking medicines dispensed in other pharmacies in the past. On the other hand, a medicine taking history of each patient is personal information. It is desired that each pharmacist cannot browse any medicine taking history at any time but can refer to a medicine taking history under certain conditions in which the necessity of referring to the medicine taking history is high.

The present disclosed technique proposes a technique in which a pharmacist can refer to a medicine taking history of each patient under certain conditions.

### MEANS FOR SOLVING THE PROBLEM

A medicine prescription support method achieved by an aspect of the present disclosed technique is a medicine prescription support method in a server which controls propriety of access to a medicine taking history through a communication network, the medicine prescription support method, executed by the server, including the steps of: receiving patient data from a user terminal; receiving prescription medicine data from the user terminal; when receiving the patient data and the prescription medicine data, setting an access propriety identifier indicating propriety of access from a pharmacy terminal to, of a database in which each patient is associated with a medicine taking history of the patient, a medicine taking history of a patient corresponding to the patient data, such that the access propriety identifier indicates permission of access; receiving a processing end signal from the pharmacy terminal; and when receiving the processing end signal, changing the access propriety identifier such that the access propriety identifier indicates non-permission of access.

Further, a medicine prescription support method achieved by an aspect of the present disclosed technique is a medicine prescription support method in a server which controls propriety of access to a medicine taking history through a communication network, the medicine prescription support method, executed by the server, including the steps of: receiving patient data from a user terminal; receiving prescription medicine data from the user terminal; receiving a processing start signal from a pharmacy terminal; when receiving the processing start signal, setting an access propriety identifier indicating propriety of access from the pharmacy terminal to, of a database in which each patient is associated with a medicine taking history of the patient, a medicine taking history of a patient corresponding to the patient data, such that the access propriety identifier indicates permission of access; receiving a processing end signal from the pharmacy terminal; and when receiving the processing end signal, changing the access propriety identifier such that the access propriety identifier indicates non-permission of access.

A medicine prescription supporting computer program achieved by an aspect of the present disclosed technique is a medicine prescription supporting computer program which causes a computer to execute each step of the above-described medicine prescription support method, so as to control access to each medicine taking history.

A medicine prescription support apparatus achieved by an aspect of the present disclosed technique is a medicine prescription support apparatus which receives a prescription through a communication network, the medicine prescription support apparatus including: a reception unit which receives patient data and prescription medicine data from a user terminal; a storage unit which stores a database in which each patient is associated with a medicine taking history of the patient; and a control unit which, when receiving the patient data and the prescription medicine data, sets an access propriety identifier indicating propriety of access from a pharmacy terminal to, of the database, a medicine taking history of a patient corresponding to the patient data such that the access propriety identifier indicates permission of access, wherein the reception unit receives a processing end signal from the pharmacy terminal, and wherein when receiving the processing end signal, the control unit changes the access propriety identifier such that the access propriety identifier indicates non-permission of access.

A medicine prescription support apparatus achieved by an aspect of the present disclosed technique is a medicine prescription support apparatus which receives a prescription through a communication network, the medicine prescription support apparatus including: a reception unit which receives patient data and prescription medicine data from a user terminal; a storage unit which stores a database in which each patient is associated with a medicine taking history of the patient; and a control unit capable of changing an access propriety identifier indicating propriety of access from a pharmacy terminal to, of the database, a medicine taking history of a patient corresponding to the patient data, wherein the reception unit receives a processing start signal and a processing end signal from the pharmacy terminal, and wherein when receiving the processing start signal, the control unit sets the access propriety identifier such that the access propriety identifier indicates permission of access, and when receiving the processing end signal, the control unit changes the access propriety identifier such that the access propriety identifiers indicates non-permission of access.

### ADVANTAGE OF THE INVENTION

According to a medicine prescription support method, a medicine prescription supporting computer program and a medicine prescription support apparatus according to the present disclosed technique, an access propriety identifier indicating propriety of access from a pharmacy terminal to a medicine taking history is changed to control the propriety of the access when predetermined conditions are satisfied, so that a pharmacist can refer to a medicine taking history of each patient when the predetermined conditions are satisfied.

### BRIEF DESCRIPTION OF THE DRAWINGS

Fig. 1 is a configuration diagram showing an example of a medicine prescription support system according to the present disclosure.
Fig. 2 is a table showing an example of a database stored in a specified pharmacy information storage unit within a user terminal according to the present disclosure.
Fig. 3 is a table showing an example of a database stored in a user information storage unit within the user terminal according to the present disclosure.
Fig. 4 is a table showing an example of a database stored in a prescription information storage unit according to the present disclosure.
Fig. 5 is a table showing the example of the database stored in the prescription information storage unit according to the present disclosure, the view showing a part following Fig. 4.
Fig. 6 is a table showing an example of a database of registered user information stored in a registered user information storage unit within a server according to the present disclosure.
Fig. 7 is a table showing an example of a database of registered pharmacist information stored in a registered pharmacist information storage unit within the server according to the present disclosure.
Fig. 8 is a table showing an example of a database stored in a medicine taking history information storage unit within the server according to the present disclosure.
Fig. 9 is a table showing an example of a database stored in a browsing right control information storage unit within the server according to the present disclosure.
Fig. 10 is a table showing an example of a database stored in a pharmacist information storage unit within a pharmacy terminal according to the present disclosure.
Fig. 11 is a processing flow chart in the medicine prescription support system according to the present disclosure.
Fig. 12 is a view showing an example of an acceptance processing screen in the pharmacy terminal according to the present disclosure.
Fig. 13 is a view showing an example of a medicine taking history information display screen in the pharmacy terminal according to the present disclosure.

### MODE FOR CARRYING OUT THE INVENTION

An embodiment showing an example of a medicine prescription support method, a medicine prescription supporting computer program and a medicine prescription support apparatus according to the present disclosure will be described below in detail with reference to the drawings.

### (Embodiment 1)

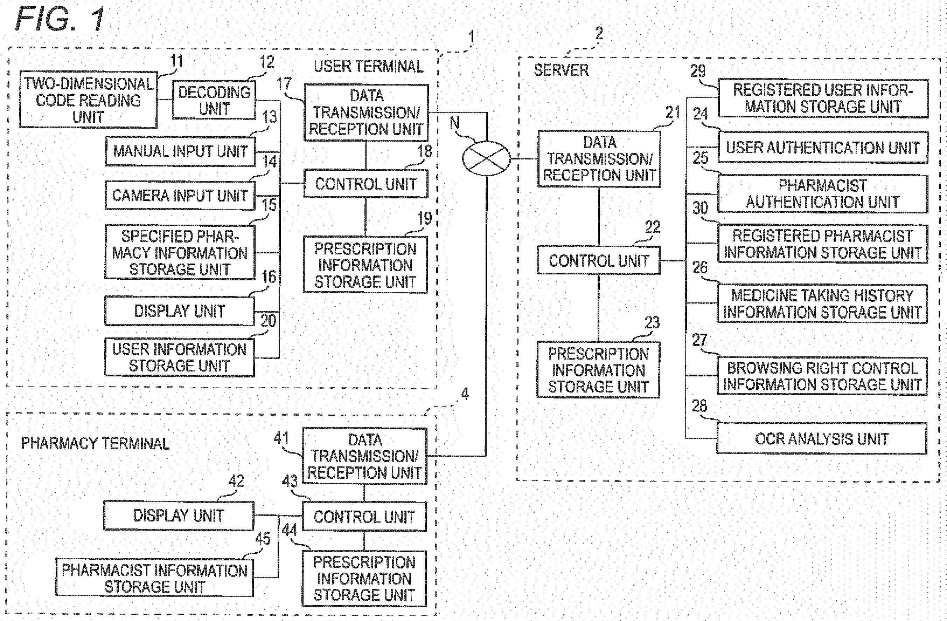

Fig. 1 is a configuration diagram showing an example of a medicine prescription support system according to the present disclosure. The medicine prescription support system is constituted by a user terminal 1, a server 2 and a pharmacy terminal 4. The user terminal 1 and the pharmacy terminal 4 are connected to the server 2 through a communication network N individually. The user terminal 1 is a terminal that is operated by a user who has received a prescription prescribed by a medical institution such as a hospital. The user terminal 1 may be, for example, a portable terminal that can be carried by the user. The user terminal 1 may be, for example, used by a patient who has used a hospital or used by a protector of the patient. The pharmacy terminal 4 is a terminal placed in a pharmacy.

The user terminal 1 has a two-dimensional code reading unit 11, a decoding unit 12, a manual input unit 13, a camera input unit 14, a specified pharmacy information storage unit 15, a display unit 16, a data transmission/reception unit 17, a control unit 18, a prescription information storage unit 19, and a user information storage unit 20.

The two-dimensional reading unit 11 reads two-dimensional codes written in a prescription. In the two-dimensional codes, a part or all of information written in the prescription has been encoded. The decoding unit 12 decodes the two-dimensional codes and converts the two-dimensional codes into electronic information. The manual input unit 13 is an input unit through which prescription information can be input manually. The manual input unit 13 is, for example, an operation button, a touch panel, etc.

The camera input unit 14 takes a picture of a prescription in which no two-dimensional code has been written. Incidentally, the two-dimensional code reading unit 11, the manual input unit 13 or the camera input unit 14 is a unit for acquiring data written in a prescription. At least one of the two-dimensional code reading unit 11, the manual input unit 13 or the camera input unit 14 may be provided.

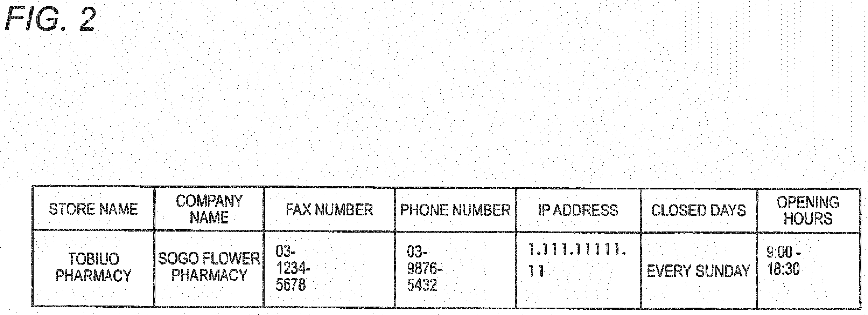

The specified pharmacy information storage unit 15 stores pharmacy data as information about a pharmacy that can be specified by a user. Information of the specified pharmacy is input from the two-dimensional code reading unit 11, the manual input unit 13, the camera input unit 14 or the data transmission/reception unit 17. The information of the specified pharmacy may be received as data through the communication network N from a database stored on the Internet or the server 2. Fig. 2 is a table showing an example of a database stored in the specified pharmacy information storage unit 15. In the specified pharmacy information storage unit 15, a store name, a company name, a FAX number, a phone number, an IP address, closed days, and opening hours are stored as the pharmacy data. However, the data are not limited to those items, but it will go well if the data include information that can specify a pharmacy uniquely.

The display unit 16 displays information of prescription read out or a result of data transmitted/received to/from the server 2. The data transmission/reception unit 17 transmits/receives data to/from the server 2 and the pharmacy terminal 4. The control unit 18 controls those processings.

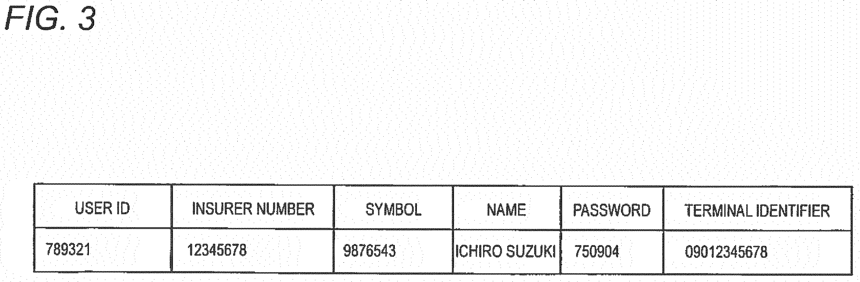

The user information storage unit 20 stores user information that can be used for authentication of a user who wants to use this system. The user information is input from the two-dimensional code reading unit 11, the manual input unit 13, the camera input unit 14 or the data transmission/reception unit 17. Fig. 3 is a table showing an example of a database stored in the user information storage unit 20. In the user information storage unit 20, a user ID, an insurer number, a symbol, a name, a password, and a terminal identifier are stored as user data. However, the data are not limited to those items, but it will go well if the data include information that can specify a user or a user terminal uniquely.

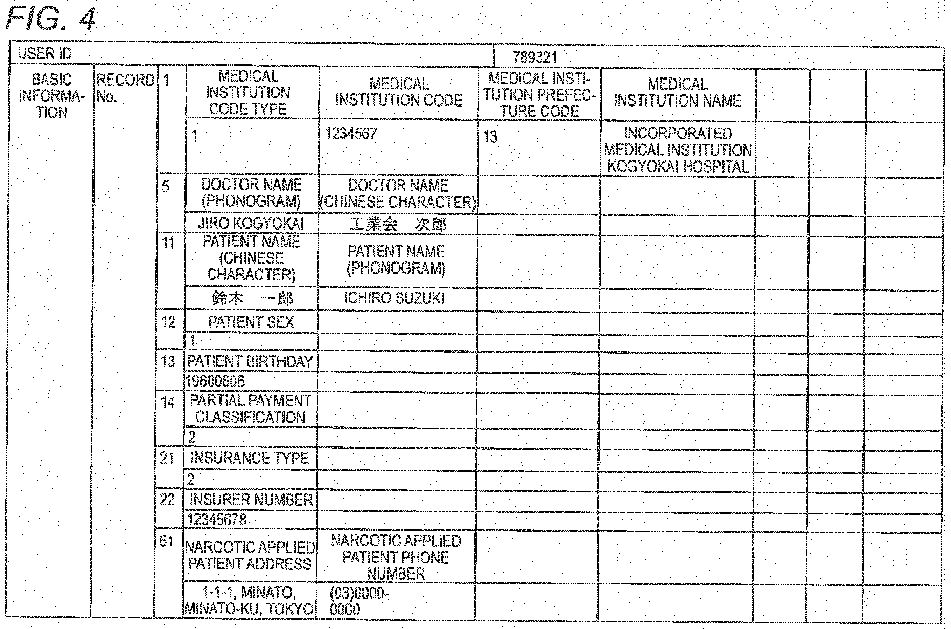

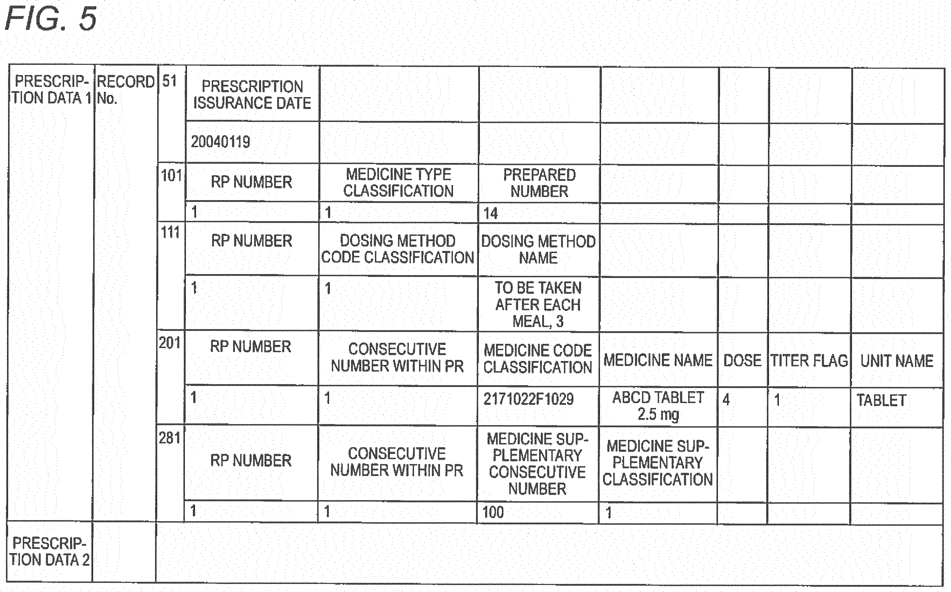

The prescription information storage unit 19 stores prescription information read out or prescription information resulting from transmission/reception to/from the server 2. Fig. 4 and Fig. 5 are tables showing an example of a database stored in the prescription information storage unit 19. The database includes basic data and prescription medicine data. Those data are stored in relation with the user ID. Fig. 4 and Fig. 5 show a database created based on a prescription issued once for a patient. When a prescription is issued once, basic data in Fig. 4 and prescription medicine data in Fig. 5 are created individually. The prescription medicine data in Fig. 5 are information following the basic data in Fig. 4. The data in Fig. 4 and the data in Fig. 5 are stored together as a single database. In addition, this database is an example of a database stored when prescription information is read out by two-dimensional codes printed in a prescription. The two-dimensional codes printed in the prescription include basic data and prescription medicine data. The basic data and the prescription medicine data belonging to the two-dimensional codes are stored in the database. Each record No. (number) designates a major category of an item. For example, the record No. 1 designates a medical institution record; No. 5, a doctor record; No. 11, a patient name record; No. 12, a patient sex record; No. 13, a patient birthday record; No. 14, a patient partial payment classification record; No. 21, an insurance type record; No. 22, an insurer number record; and No. 61, a narcotic application record. Categories in the medical institution record include minor category items such as a medical institution code type, a medical institution code, a medical institution prefecture code, a medical institution name, etc. For example, a predetermined number such as 1 designating a medical department, 3 designating a dental department, and 6 designating a visit is recorded in the medical institution code type. The medical institution code is a code with which a receipt should be submitted. The medical institution prefecture code is a number assigned to each prefecture. Chinese characters (kanji) of a medical institution name are recorded in the medical institution name. Items of the basic data are not limited to those items. The prescription medicine data are stored for every individual medicine written in a prescription. The record No. 51 is a prescription issuance date record; No. 101, a medicine type record; No. 111, a dosing method record; No. 201, a medicine record; and No. 281, a medicine supplementary record. Each record further includes minor category items. The prescription medicine data are not limited to those items.

In addition, a part of data may be acquired manually or by reading with an OCR.

Incidentally, "ABCD tablet" is written in the medicine record No. 201. In fact, a specific tradename of a medicine is written in the alphabetical part of "ABCD". The tradename may include a registered tradename. A similar part in another drawing may be interpreted in the same manner.

The server 2 has a data transmission/reception unit 21, a control unit 22, a prescription information storage unit 23, a user authentication unit 24, a registered user information storage unit 29, a pharmacist authentication unit 25, a registered pharmacist information storage unit 30, a medicine taking history storage unit 26, a browsing right control information storage unit 27, and an OCR analysis unit 28. The server 2 functions as a medicine prescription support apparatus that can receive a prescription through the communication network N.

The data transmission/reception unit 21 transmits/receives data to/from the user terminal 1 and the pharmacy terminal 4. The control unit 22 controls processings entirely. In addition, the control unit 22 rewrites the browsing right control information storage unit 27. The prescription information storage unit 23 accumulates prescription information sent from the user terminal 1. A database recorded in the prescription information storage unit 23 is similar to that in Fig. 4 and Fig. 5.

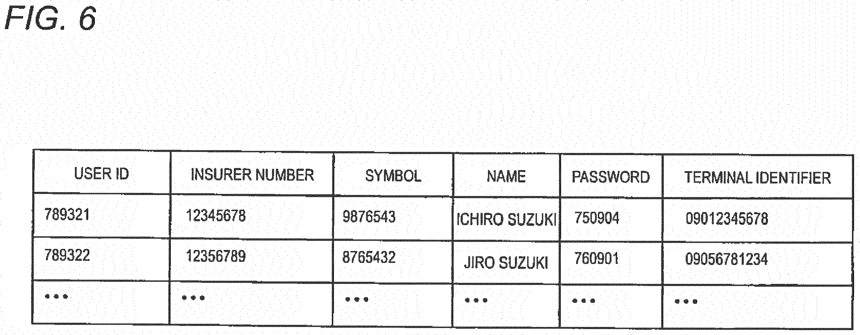

The registered user information storage unit 29 stores a list of users who can use this system. Fig. 6 is a table showing an example of a database of registered user information stored in the registered user information storage unit 29. In the database of the registered user information, a user ID, an insurer number, a symbol, a name, a password and a terminal identifier are stored as registered user data for each user. The registered user data are not limited to those items, but it will go well if the data can identify each user.

The user authentication unit 24 checks user data received from the user terminal 1 with the database stored in the registered user information storage unit 29, so as to check whether the user who has transmitted information from the user terminal 1 is a registered user or not.

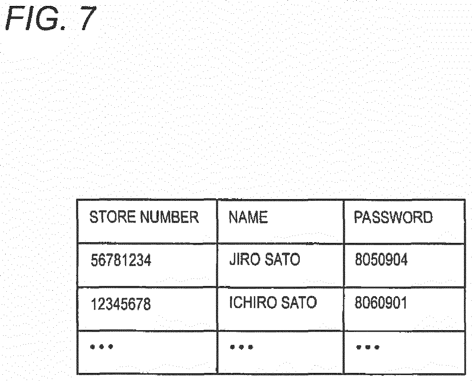

The registered pharmacist information storage unit 30 stores a list of pharmacists who can use this system. Fig. 7 is a table showing an example of a database of registered pharmacist information stored in the registered pharmacist information storage unit 30. In the database of the registered pharmacist information, a store number, a name and a password are stored as registered pharmacist data for each pharmacist. The store number is a number of a store the pharmacist belongs to. The registered pharmacist data are not limited to those items, but it will go well if the data can identify each pharmacist.

The pharmacist authentication unit 25 checks pharmacist data received from the pharmacy terminal 4 with the database stored in the registered pharmacist information storage unit 30, so as to check whether the pharmacist who has transmitted information from the pharmacy terminal 4 is a registered pharmacist or not.

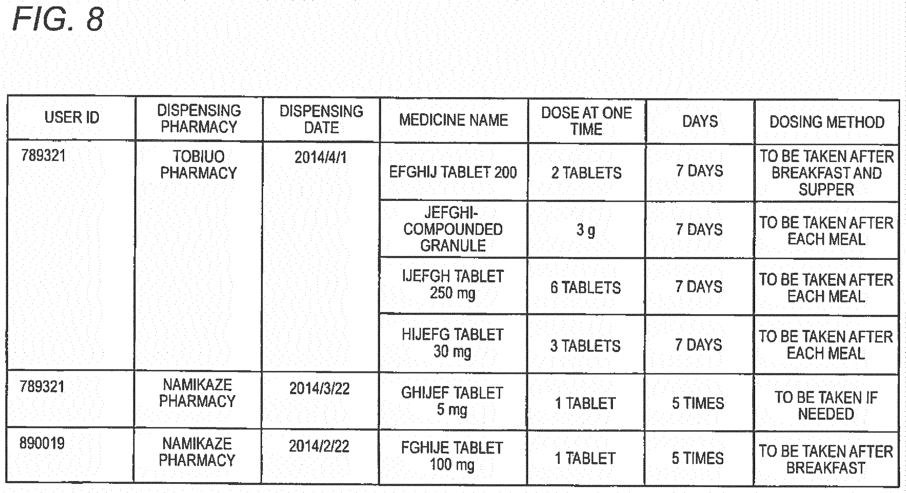

The medicine taking history information storage unit 26 accumulates information of past medicine taking histories of patients. Fig. 8 is a table showing an example of a database stored in the medicine taking history information storage unit 26. In the medicine taking history information, medicines dispensed by dispensing pharmacies and purchased by users in the past are stored as past medicine taking history information for each patient. Here, a user ID, a dispensing pharmacy name, a dispensing date, a medicine name, a dose at one time, days, and a dosing method are stored in each entry.

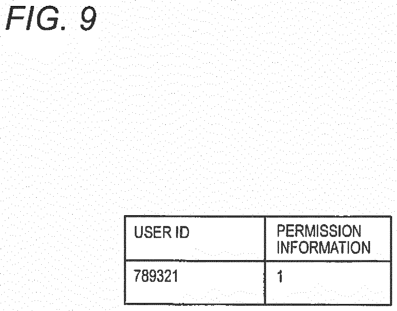

The browsing right control information storage unit 27 stores information for controlling the right to browse the medicine taking history information. Fig. 9 is a table showing an example of a database stored in the browsing right control information storage unit 27. An access propriety identifier indicating propriety of access from the pharmacy terminal 4 is stored as permission information for each user ID. For example, the access propriety identifier may be set as "I" when access from the pharmacy terminal 4 is permitted, and as "0" when access from the pharmacy terminal 4 is not permitted. Rewriting the access propriety identifier is controlled and executed by the control unit 22.

The OCR analysis unit 28 analyzes a camera photographed image of a prescription transmitted from the user terminal 1 and converts the image into electronic information. The OCR analysis unit 28 may be removed when the user terminal 1 is not provided with the camera input unit 14.

The pharmacy terminal 4 has a data transmission/reception unit 41, a control unit 43, a prescription information storage unit 44, a display unit 42 and a pharmacist information storage unit 45. The data transmission/reception unit 41 transmits/receives data to/from the user terminal 1. The control unit 43 controls processings entirely. The prescription information storage unit 44 accumulates prescription information sent from the user terminal 1. A database recorded in the prescription information storage unit 44 is similar to that in Fig. 4 and Fig. 5.

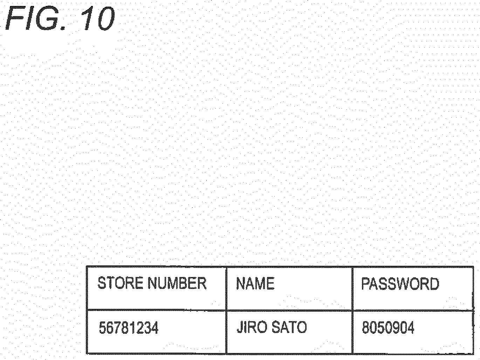

The display unit 42 displays received prescription information. The pharmacist information storage unit 45 accumulates information for authenticating each pharmacist in this system. Fig. 10 shows an example of a database stored in the pharmacist information storage unit 45. In the pharmacist information storage unit 45, a store number, a name and a password are stored as pharmacist data. The data are not limited to those items, but it will go well if the data include information for identifying a pharmacist uniquely.

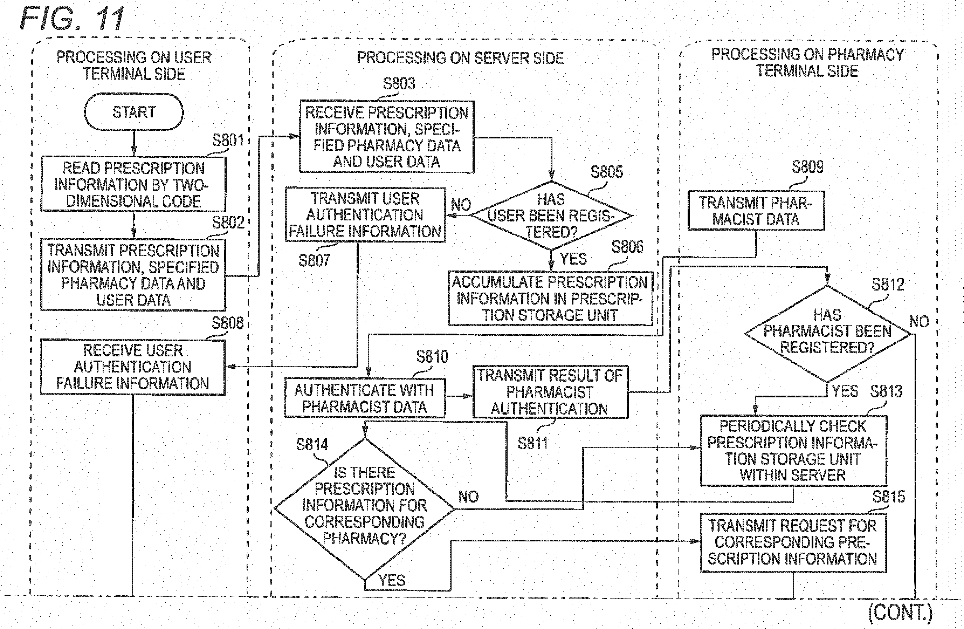

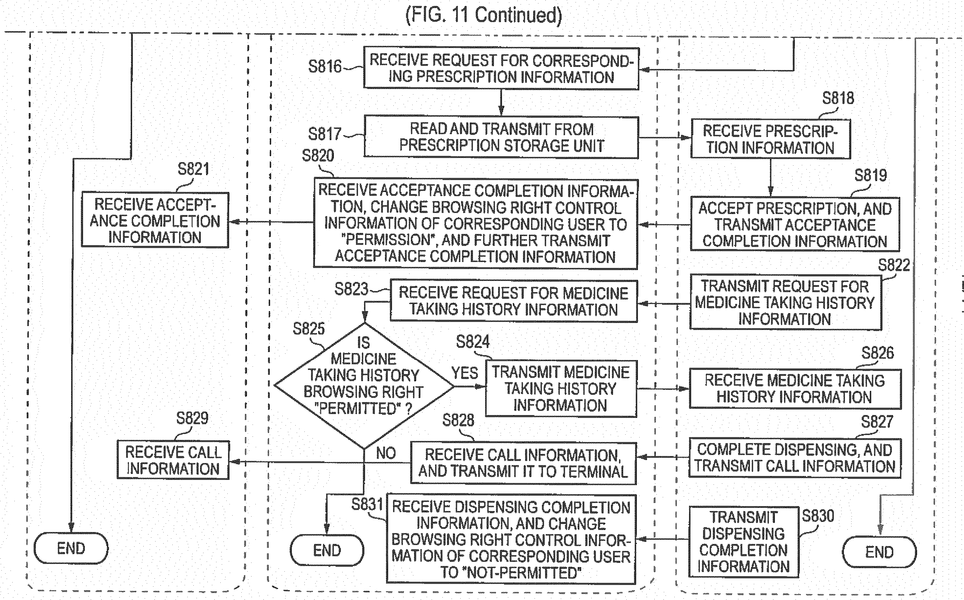

A processing flow to be executed by each configuration will be described below. Fig. 11 is a chart showing an example of a processing flow in the medicine prescription support system disclosed herein. Here, an example will be shown about the case where prescription information is read by the two-dimensional code reading unit 11 of the user terminal 1.

First, the two-dimensional code reading unit 11 reads out prescription information from two-dimensional codes. The decoding unit 12 decodes the prescription information read out and converts the prescription information into electronic data. As a result, the electric data are accumulated in the prescription information storage unit 19 (Step S801).

In addition to the electronic data, the control unit 18 reads out pharmacy data of a specified pharmacy from the specified pharmacy information storage unit 15 and reads out user data from the user information storage unit 20. The prescription information, the pharmacy data and the user data are transmitted to the server 2 through the data transmission/reception unit 17 (Step S802). The data transmission/reception unit 21 of the server 2 receives the prescription information, the pharmacy data and the user data (Step S803). The prescription information, the pharmacy data and the user data may be transmitted and/or received individually and separately or may be transmitted and/or received as a block of data simultaneously through one time of communication. According to one example in which the prescription information, the pharmacy data and the user data are transmitted as a block of data simultaneously through one time of communication, when a user pushes down, for example, a button indicating data transmission, for example, by use of the manual input unit 13 in the user terminal 1, the control unit 18 transmits the prescription information, the pharmacy data and the user data to the server 2 through the communication network N set in a communication state.

Under the control of the control unit 22, the user authentication unit 24 authenticates whether the user is a registered user or not based on the user data. Here, it is verified whether the user ID of the received user data coincides with a user ID stored in the database of the registered user information or not and it is verified whether the password of the received user data coincides with a password associated with the user ID or not (Step S805). When the user has been registered (Yes in Step S805), the prescription information is accumulated in the prescription information storage unit 23 together with the user data (Step S806). On the other hand, when the user has not been registered (No in Step S805), the data transmission/reception unit 21 transmits user authentication failure information to the user terminal 1 (Step S807). In the user terminal 1, the data transmission/reception unit 17 receives the user authentication failure information (Step S808).

When a pharmacist uses the pharmacy terminal 4, an ID and a password of a terminal user are received. This is, for example, based on user authentication in a typical PC. On success in the user authentication, an application begins to operate. Thus, in the pharmacy terminal 4, the control unit 43 reads out pharmacist data from the pharmacist information storage unit 45 and transmits the pharmacist data to the server 2 through the data transmission/reception unit 41 (Step S809).

In the server 2, the data transmission/reception unit 21 receives the pharmacist data transmitted from the pharmacy terminal 4, and the control unit 22 makes the pharmacist authentication unit 25 check whether the pharmacist has been registered or not (Step S810). The data transmission/reception unit 21 transmits the checking result to the pharmacy terminal 4 (Step S811). Whether the pharmacist has been registered or not is checked by determination as to whether the received pharmacist data coincide with registered pharmacist data stored in the database of the registered pharmacist information storage unit 30 or not.

In the pharmacy terminal 4, the data transmission/reception unit 41 receives the checking result as to whether the pharmacist has been registered or not, and the control unit 43 checks the authentication result (Step S812). As long as the pharmacist has been registered (Yes in Step S812), prescription information accumulated in the server 2 is confirmed periodically (Step S813). Here, the control unit 43 transmits the pharmacist data including pharmacy data to the server 2 through the data transmission/reception unit 41, and the control unit 22 checks the prescription information storage unit 23 to confirm whether unprocessed prescription information for the pharmacy has been accumulated or not (Step S814). Whether prescription information has been unprocessed or not may be determined by determination as to whether data has been stored since the last confirmation time based on a data storage time stored in advance in the database in relation with a prescription.

When unprocessed prescription information for the pharmacy has not been accumulated (No in Step S814), the result is transmitted to the data transmission/reception unit 41 of the pharmacy terminal 4. The control unit 43 performs Step S813 again after a certain time. On the other hand, when unprocessed prescription information for the pharmacy has been accumulated (Yes in Step S814), the control unit 43 transmits a request for the corresponding prescription information to the server 2 (Step S815).

On receiving the request through the data transmission/reception unit 21 (Step S816), the control unit 22 of the server 2 reads out the prescription information from the prescription information storage unit 23 and transmits the prescription information to the pharmacy terminal 4 (Step S817).

The pharmacy terminal 4 receives the prescription information (Step S818), receives the prescription to accumulate the prescription information in the prescription information storage unit 44, and transmits an acceptance completion signal (processing start signal) to the server 2 (Step S819).

In the server 2, when the data transmission/reception unit 21 receives the acceptance completion signal (processing start signal), the control unit 22 changes, of the browsing right control information in the browsing right control information storage unit 27, an identifier stored in a permission information field for a corresponding user so that the identifier can designate browsing permission. Further, the control unit 22 transmits an acceptance completion signal to the user terminal 1 (Step S820). On the pharmacy side, when the prescription information is checked, acceptance may be refused because there is no stock. Therefore, processing of acceptance is performed thus in the pharmacy terminal 4. On receiving the processing start signal from the pharmacy terminal 4, the server 2 changes the browsing right control information so that browsing permission can be given only to the pharmacy terminal 4 that will dispense medicines really. As described above, as soon as the server 2 receives the processing start signal, the server 2 changes the browsing right control information and also performs another processing. The processing start signal is not limited to the acceptance completion signal. It will go well if the processing start signal is a signal serving as a trigger for the server 2 to change the browsing right control information and also perform another processing as soon as the server 2 receives the processing start signal.

In addition, the server 2 has a status database in which each user is associated with the acceptance status of the user for each pharmacy. The server 2 registers user data received in Step S803, for example, a user ID in the status database in advance. On receiving an acceptance completion signal in Step S820, the control unit 22 changes the status database so that the status of the user can designate acceptance completion in relation with the user ID. The pharmacy terminal 4 reads out the status database periodically and displays the status on the display unit 42.

In the user terminal 1, the data transmission/reception unit 17 receives the acceptance completion signal, and notifies the user of the completion of acceptance on the display unit 16 (Step S821).

Incidentally, here, on receiving the acceptance completion signal (processing start signal), the server 2 changes the browsing right control information into a state indicating browsing permission. However, in the stage where the server 2 has received the prescription medicine data and at least one of the basic data and the user data in Step S803, the received data may be regarded as the processing start signal, and the browsing right control information may be changed into the state indicating browsing permission. Each of the basic data and the user data are defined as patient data including personal information for identifying a patient. When at least the patient data and the prescription medicine data are received, the browsing right control information is changed. In addition, as described in Step S805, on receiving the prescription medicine data, the basic data and the user data, the server 2 advances to the step in which it is authenticated whether the user is a registered user or not. It can be therefore said that to receive the patient data and the prescription medicine data is to receive a signal serving as a trigger to change the browsing right control information and also perform another processing.

Next, in the pharmacy, dispensing work is started based on the accepted prescription. Here, the pharmacist must check medicines the corresponding user has taken till now or is taking now, and check a combination to be avoided or occurrence of duplicated preparation of medicines.

Therefore, the pharmacist browses the medicine taking history of the corresponding user. Specifically, the pharmacist operates the pharmacy terminal 4, for example, to execute input for browsing the medicine taking history. In response to the input, the control unit 43 transmits a medicine taking history information request to the server 2 through the data transmission/reception unit 41 (Step S822). The input to the pharmacy terminal 4 is performed through an input unit (not shown) controlled by the control unit 43.

When the data transmission/reception unit 21 of the server 2 receives the medicine taking history information request transmitted from the pharmacy terminal 4 (Step S823), the control unit 22 refers to the browsing right control information storage unit 27 and checks whether the access propriety identifier is an identifier for permitting access from the pharmacy terminal 4 (Step S825). Here, when the identifier "permits" the access (Yes in Step S825), that is, only when the part of permission information in the aforementioned example of the database of the browsing right control information storage unit 27 is "1", medicine taking history information of the corresponding user is read out from the medicine taking history information storage unit 26, and the medicine taking history information of the user is transmitted to the pharmacy terminal 4 through the data transmission/reception unit 21 (Step S824). The data transmission/reception unit 41 of the pharmacy terminal 4 receives the medicine taking history information of the user and the control unit 43 displays the medicine taking history information of the user on the display unit 42 (Step S826).

Next, when the pharmacist has dispensed medicines with reference to the medicine taking history information, the control unit 43 transmits a call signal to the server 2 through the data transmission/reception unit 41 in order to call the user to come to the pharmacy to pick up the medicines (Step S827). The data transmission/reception unit 21 receives the call signal and transmits a patient call signal to the user terminal (Step S828).

In addition, when the server 2 receives the call signal in Step S828, the control unit 22 changes the status database which belongs to the server 2 and in which each user is associated with the acceptance status of the user, so that the status of the user can indicate the user is under calling. The pharmacy terminal 4 reads out the status database periodically and displays the status on the display unit 42.

The user terminal 1 receives the patient call signal through the data transmission/reception unit 17, and the control unit 18 displays, on the display unit 16, that the user is called from the pharmacy (Step S829).

The user knows the medicines have been prepared. The user brings the original prescription to the pharmacy accordingly in order to call for the medicines. In the pharmacy, the pharmacist deals with the user and delivers the medicines in exchange for the original prescription. The user makes a payment.

After that, the pharmacist performs dispensing completion processing in the pharmacy terminal 4 at the end of a series of processings. Specifically, under the control of the control unit 43, the data transmission/reception unit 41 transmits a dispensing completion signal (processing end signal) to the server 2 together with the user data (Step S830). In addition, when the dispensing completion processing is performed, the control unit 43 reflects and displays the dispensing completion on the display unit 42 of the pharmacy terminal 4. On this occasion, the prescription information including prescription medicine data about the medicines prepared really may be transmitted to the server 2. Medicines prescribed by a doctor may be changed into and prepared as generic products or the like. Then data are stored as medicine taking history information in the medicine taking history information storage unit 26 of the server 2. The prescription information including the prescription medicine data of the medicines prepared really is not transmitted to the server 2 in Step S830 but two-dimensional codes of the prescription information including the prescription medicine data of the medicines prepared really may be printed in a description for the dispensed medicines when the medicines are delivered to the user. In this case, the user reads out the two-dimensional codes printed in the description for the dispensed medicines through the two-dimensional code reading unit 11 of the user terminal I, and stores the read contents as medicine taking history information in the prescription information storage unit 19. The user transmits the information to the server 2 to update the medicine taking history storage unit 26.

In the server 2, the data transmission/reception unit 21 receives the dispensing completion signal (processing end signal). On receiving the processing end signal, the control unit 22 changes, of the browsing right control information in the browsing right control information storage unit 27, the permission information for the corresponding user so that the permission information can indicate non-permission of browsing (Step S831).

That is, in the aforementioned example of the database of the browsing right control information storage unit 27, the part of the permission information is set as "0". Alternatively, the information (such as the user ID) of the corresponding user registered in the browsing right control information storage unit 27 may be deleted. In this case, when the server 2 receives user data in Step S803, the ID of the user is stored in the browsing right control information storage unit 27, and the permission information is set in a state indicating non-permission of browsing initially. When predetermined conditions as described above are satisfied, the permission information is changed into a state indicating permission of browsing. Then in Step 5831, the user ID and the permission information registered in the browsing right control information storage unit 27 are deleted.

For example, after Step S831, that is, after the browsing right control information storage unit 27 is changed into "0", the pharmacy terminal 4 cannot gain access to any medicine taking history information even if the server 2 receives a medicine taking history information request from the pharmacy terminal 4.

In addition, when the server 2 receives the dispensing completion signal in Step S831, the control unit 22 changes the status database which belongs to the server 2 and in which each user is associated with an acceptance status of the user, so that the status of the user can indicate completion of dispensing. The pharmacy terminal 4 reads out the status database periodically and displays the status on the display unit 42.

Here, when the server 2 receives the dispensing completion signal from the pharmacy terminal 4, the server 2 regards the received dispensing completion signal as the processing end signal and changes the browsing right control information into a state designating non-permission of browsing. However, the call signal transmitted to the server 2 from the pharmacy terminal in Step S827 may be regarded as the processing end signal. That is, when the server 2 receives the call signal from the pharmacy terminal 4 in Step S828, the control unit 22 may change, of the browsing right control information in the browsing right control information storage unit 27, the permission information of the corresponding user into a state indicating non-permission of browsing. As described above, when the call signal is regarded as the processing end signal, the server 2 transmits a patient call signal to the user terminal 1 when receiving the call signal, while the control signal 22 changes the browsing right control information. When the dispensing completion signal is regarded as the processing end signal, the server 2 changes the status database belonging to the server 2 when receiving the dispensing completion signal, while the control unit 22 changes the browsing right control information. That is, it will go well if the processing end signal is a signal serving as a trigger with which the server 2 changes the browsing right control information and also performs another processing.

Incidentally, here, when the two-dimensional codes are absent from the prescription, in place of Step S801, a prescription image photographed by the camera input unit 14 may be transmitted to the server 2 through the data transmission/reception unit 17, and the OCR analysis unit 28 may perform OCR analysis on the image and convert the image into electronic information.

In addition, in the same manner, when the two-dimensional codes are absent from the prescription, information input manually through the manual input unit 13 may be transmitted to the server 2 through the data transmission/reception unit 17 in place of Step S801.

Incidentally, a display for confirmation as to whether the user agrees with disclosure of the medicine taking history information may be displayed on the display unit 16 in the user terminal 1. When the user gives the user terminal 1 an input indicating that the user agrees with disclosure of the medicine taking history information, a user agreement signal is transmitted from the user terminal 1 to the server 2. When the server 2 receives the user agreement signal, the server 2 changes the browsing right control information. Also in this case, Step S822 to Step S826 are executed in the same manner. In addition, processing from Step S830 to Step S831 is executed in the same manner. That is, a medicine prescription support method described herein is a medicine prescription support method in a server controlling propriety of access from a pharmacy terminal to data of a medicine taking history of each patient accumulated in the server. The pharmacy terminal and a user terminal make communication with the server through a communication network. The server executes the steps of: receiving a user agreement signal from the user terminal on receiving the user agreement signal, setting an access propriety identifier indicating propriety of access from the pharmacy terminal to data of a medicine taking history of the user, so that the access propriety identifier can indicate permission of access; receiving a processing end signal from the pharmacy terminal; and on receiving the processing end signal, changing the access propriety identifier so that the access propriety identifier can indicate non-permission of access. In addition, a medicine prescription support apparatus described herein is a medicine prescription support apparatus that can be constituted by the aforementioned server 2. The medicine prescription support apparatus makes communication with a pharmacy terminal and a user terminal through a communication network and includes: a reception unit that receives a user agreement signal from the user terminal; a storage unit that stores a database in which each patient is associated with a medicine taking history of the patient; and a control unit that can change an access propriety identifier indicating propriety of access from the pharmacy terminal to data of a medicine taking history of the user. The reception unit receives a processing end signal from the pharmacy terminal. When the reception unit receives the user agreement signal, the control unit sets the access propriety identifier so that the access propriety identifier can indicate permission of access. When the reception unit receives the processing end signal, the control unit changes the access propriety identifier so that the access propriety identifier can indicate non-permission of access.

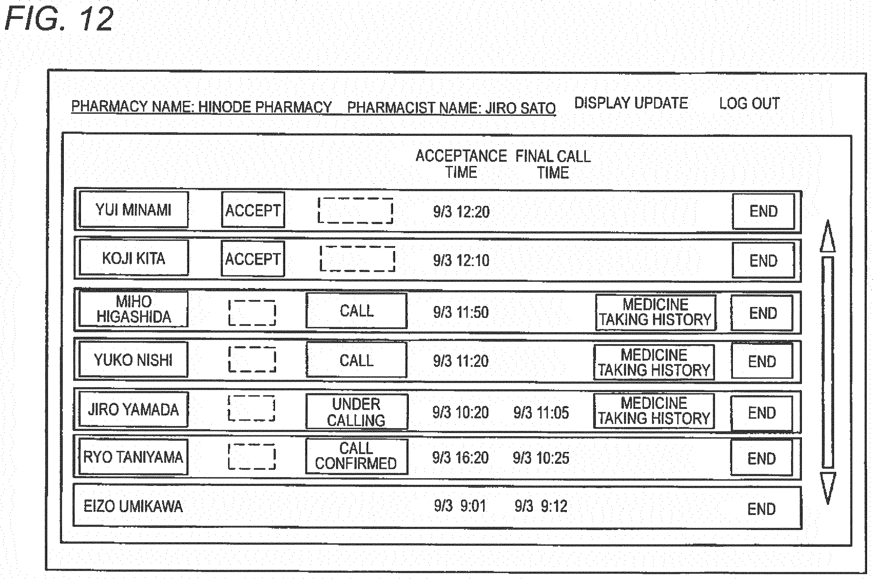

Fig. 12 is a view showing an example of a screen to be displayed on the pharmacy terminal 4 in Steps S818, S819, S822 and S827.

In the pharmacy, a screen as shown in Fig. 12 is displayed on the display unit, on which processing for accepting a prescription to be transmitted to the pharmacy can be advanced. On the screen, users to be processed are displayed individually. Each user displayed with an "accept" button is a user whose prescription information has been received from the server in Step S818 and who will be accepted from now. Each user displayed with a "call" button is a user whose medicines are being dispensed now and who will be called as soon as the medicines are dispensed. When the call button is pushed in Step S827, a call signal is transmitted. When the "call" button is pushed, the button is changed into a display of "under calling".

In addition, a "medicine taking history" button shows that medicine taking history information of the corresponding user will be displayed when the "medicine taking history" button is pushed. The "medicine taking history" button may be always displayed visually so that the "medicine taking history" button can be pushed at any time. In this case, when the button is pushed, the server 2 may check whether a medicine taking history information browsing right of the corresponding user is "permitted". The medicine taking history information browsing right of the user may be displayed only when it is "permitted", that is, when the part of permission information in the example of the database of the browsing right control information storage unit 27 is "1".

In addition, in consideration of convenience for a user of the pharmacy terminal 4, the pharmacy terminal 4 may check at a certain interval whether a medicine taking history information browsing right of a user corresponding to each "medicine taking history" button is "permitted". When the medicine taking history information browsing right is "permitted", the "medicine taking history" button may be displayed so that it can be pushed down.

The pharmacy terminal 4 periodically reads out the status database stored in the server 2 and displays the status on the display unit 42. However, the aforementioned checking of the medicine taking history information browsing right at a certain interval may be replaced by the display information update timing of the status information displayed on the pharmacy terminal 4.

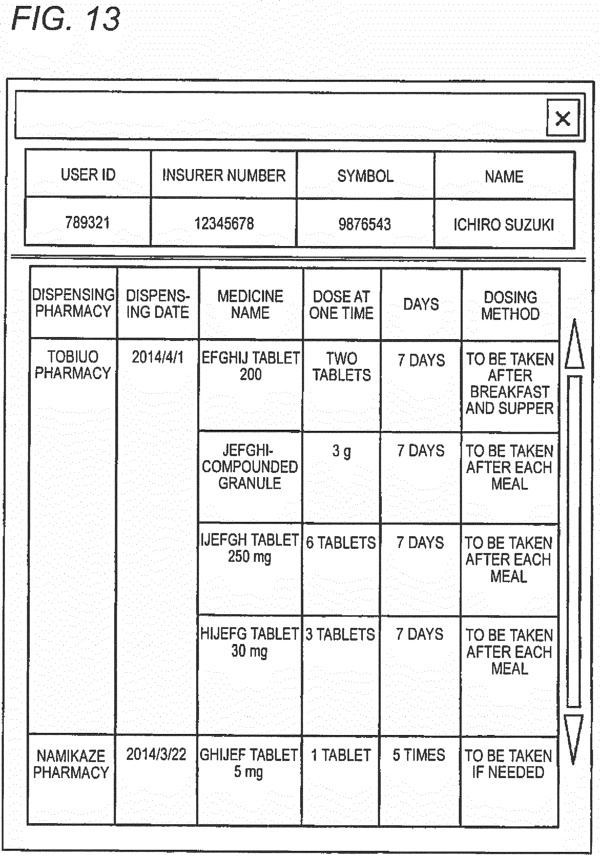

Fig. 13 is a view showing an example of a screen displayed on the pharmacy terminal 4 in Step S826, in which the medicine taking history information of a user is displayed as an image. A medicine taking history including dispensing pharmacy names, dates and hours, medicine names, etc. is displayed together with information of the corresponding user stored in the registered user information storage unit 29.

Due to the medicine taking history information storage unit 26 and the browsing right control information storage unit 27 provided thus, when a user sends prescription information to a pharmacy and the pharmacy receives preparation of medicines, permission of browsing a medicine taking history as privacy information can be presented to limited persons who can browse the medicine taking history and within a limited period without any special action of the user.

When the technique disclosed herein is used, medicine taking histories can be managed electronically, for example, by an application for a smartphone in place of a method for managing medicine taking histories on paper media in the background art. The usability for users can be improved. In addition, when medicine taking histories can be referred to through a network, a pharmacy terminal can gain access to a medicine taking history of each patient. Thus, it is also unnecessary to show, directly to a pharmacist, a user terminal in which a medicine taking history has been stored. For example, assume that a user sends a prescription from a user terminal to a pharmacy in advance and the pharmacy receives the prescription. When the user comes to the pharmacy to receive medicines after the medicines have been prepared, a pharmacist can confirm a medicine taking history of a patient from a pharmacy terminal. The pharmacist can refer to the medicine taking history of the patient unless the patient delivers, to the pharmacy, a medicine taking history book on a paper medium or a user terminal storing a medicine taking history. That is, each medicine taking history is managed in a server so that the medicine taking history can be browsed from a pharmacy. However, if the pharmacy can view the medicine taking history as privacy information without permission or if the pharmacy permitted to browse the medicine taking history can view the medicine taking history indefinitely, there will be a problem for the user. By use of the technique disclosed herein, it is possible to control the period in which the pharmacy can gain access to the medicine taking history. For example, when the intention of a user to permit access to his/her medicine taking history is expressed as an action such as pushing down a button, the user hardly decides the timing of terminating the browsing permission because the user does not know the timing when the pharmacist terminates dispensing work. On the other hand, according to the technique disclosed herein, the propriety of access to the medicine taking history is controlled by the server so that the timing of terminating browsing the medicine taking history can be controlled. In addition, the period in which the medicine taking history can be browsed can be controlled without any special operation of the user.

Incidentally, the invention is not limited to the aforementioned embodiment, but changes, improvements, etc. can be made suitably. In addition, materials, shapes, dimensions, numbers, forms, arrangement places, etc. of constituent elements in the aforementioned embodiment are not limited but may be selected desirably if the invention can be attained.

The present application is based on a Japanese patent application (Japanese Patent Application No. 2014-127317) filed on June 20, 2014, the contents of which are incorporated herein by reference.

### INDUSTRIAL APPLICABILITY

According to the technique disclosed herein, the propriety of access to each medicine taking history is controlled so that a pharmacist can refer to a medicine taking history of each patient when predetermined conditions are satisfied.

### DESCRIPTION OF REFERENCE NUMERALS AND SIGNS

- 1:: User Terminal
- 2:: Server (Medicine Prescription Support Apparatus)
- 4:: Pharmacy Terminal
- 11:: Two-Dimensional Code Reading Unit
- 12:: Decoding Unit
- 13:: Manual Input Unit
- 14:: Camera Input Unit
- 15:: Specified Pharmacy Information Storage Unit
- 16:: Display Unit
- 17:: Data Transmission/Reception Unit
- 18:: Control Unit
- 19:: Prescription Information Storage Unit
- 20:: User Information Storage Unit
- 21:: Data Transmission/Reception Unit
- 22:: Control Unit
- 23:: Prescription Information Storage Unit
- 24:: User Authentication Unit
- 25:: Pharmacist Authentication Unit
- 26:: Medicine Taking History Information Storage Unit
- 27:: Browsing Right Control Information Storage Unit
- 28:: OCR Analysis Unit
- 29:: Registered User Information Storage Unit
- 30:: Registered Pharmacist Information Storage Unit
- 41:: Data Transmission/Reception Unit
- 42:: Display Unit
- 43:: Control Unit
- 44:: Prescription Information Storage Unit
- 45:: Pharmacist Information Storage Unit
- N:: Communication Network

## Claims

1. A medicine prescription support method in a server which controls propriety of access to a medicine taking history through a communication network, the medicine prescription support method, executed by the server, comprising the steps of:
receiving patient data from a user terminal;
receiving prescription medicine data from the user terminal;
when receiving the patient data and the prescription medicine data, setting an access propriety identifier indicating propriety of access from a pharmacy terminal to, of a database in which each patient is associated with a medicine taking history of the patient, a medicine taking history of a patient corresponding to the patient data, such that the access propriety identifier indicates permission of access;
receiving a processing end signal from the pharmacy terminal; and
when receiving the processing end signal, changing the access propriety identifier such that the access propriety identifier indicates non-permission of access.

2. A medicine prescription support method in a server which controls propriety of access to a medicine taking history through a communication network, the medicine prescription support method, executed by the server, comprising the steps of:
receiving patient data from a user terminal;
receiving prescription medicine data from the user terminal;
receiving a processing start signal from a pharmacy terminal;
when receiving the processing start signal, setting an access propriety identifier indicating propriety of access from the pharmacy terminal to, of a database in which each patient is associated with a medicine taking history of the patient, a medicine taking history of a patient corresponding to the patient data, such that the access propriety identifier indicates permission of access;
receiving a processing end signal from the pharmacy terminal; and
when receiving the processing end signal, changing the access propriety identifier such that the access propriety identifier indicates non-permission ofaccess.

3. The medicine prescription support method according to claim 1 or 2, wherein the server receives the processing end signal from the pharmacy terminal when the pharmacy terminal receives an input indicating that medicines of a prescription of the patient have been prepared.

4. The medicine prescription support method according to claim 1 or 2, wherein the server receives the processing end signal from the pharmacy terminal when the pharmacy terminal receives an input for performing processing for calling a user.

5. The medicine prescription support method according to claim 4, wherein the server transmits a call signal to the user terminal when the server receives the processing end signal.

6. The medicine prescription support method according to any one of claims 1 to 5, wherein both the patient data and the prescription medicine data are received at one time of communication between the server and the user terminal.

7. A medicine prescription supporting computer program which causes a computer to execute each step of the medicine prescription support method according to any one of claims 1 to 6, so as to control access to each medicine taking history.

8. A medicine prescription support apparatus which receives a prescription through a communication network, the medicine prescription support apparatus comprising:
a reception unit which receives patient data and prescription medicine data from a user terminal;
a storage unit which stores a database in which each patient is associated with a medicine taking history of the patient; and
a control unit which, when receiving the patient data and the prescription medicine data, sets an access propriety identifier indicating propriety of access from a pharmacy terminal to, of the database, a medicine taking history of a patient corresponding to the patient data such that the access propriety identifier indicates permission ofaccess,
wherein the reception unit receives a processing end signal from the pharmacy terminal, and
wherein when receiving the processing end signal, the control unit changes the access propriety identifier such that the access propriety identifier indicates non-permission of access.

9. A medicine prescription support apparatus which receives a prescription through a communication network, the medicine prescription support apparatus comprising:
a reception unit which receives patient data and prescription medicine data from a user terminal;
a storage unit which stores a database in which each patient is associated with a medicine taking history of the patient; and
a control unit capable of changing an access propriety identifier indicating propriety of access from a pharmacy terminal to, of the database, a medicine taking history of a patient corresponding to the patient data,
wherein the reception unit receives a processing start signal and a processing end signal from the pharmacy terminal, and
wherein when receiving the processing start signal, the control unit sets the access propriety identifier such that the access propriety identifier indicates permission of access, and when receiving the processing end signal, the control unit changes the access propriety identifier such that the access propriety identifiers indicates non-permission of access.

10. The medicine prescription support apparatus according to claim 8 or 9, wherein the reception unit receives the processing end signal from the pharmacy terminal when the pharmacy terminal receives an input indicating that medicines of a prescription of the patient have been prepared.

11. The medicine prescription support apparatus according to claim 8 or 9, wherein the reception unit receives the processing end signal from the pharmacy terminal when the pharmacy terminal receives an input for performing processing for calling a user

12. The medicine prescription support apparatus according to claim 11, further comprising a transmission unit that transmits a call signal to the user terminal when the reception unit receives the processing end signal.
